# DEMANDE DE BREVET EUROPEEN

(11) **EP 3 598 996 A1**
(43) Date de publication de la demande: **29.01.2020**
(21) Numéro de dépôt: 19188391.7
(22) Date de dépôt: 25.07.2019
(51) Int. Cl.: A61Q 3/02, A61K 8/37, A61K 8/55, A61K 8/34, A61K 8/46, A61K 8/39, A61K 8/81, A61K 8/73

(54) **COMPOSITIONS AMÉLIORÉES COMME REVÊTEMENT DE BASE APPLICABLE SUR LES ONGLES**

(30) Priorité: 27.07.2018 LU 100888
(71) Demandeur: International Lacquers S.A., 3225 Bettembourg (LU)
(72) Inventeur: VENTURA, Emmanuelle, 57000 Metz (FR); DEROSIER, Frédéric, 57680 Corny sur Moselle (FR); EDDOUMY, Fatima, 57840 Ottange (FR)
(74) Mandataire: Office Freylinger

(57) **Abrégé**

Composition de revêtement de base pour les vernis à ongles comprenant au moins un solvant organique cosmétiquement acceptable, au moins un agent filmogène, au moins un système de photoréticulation, au moins un monomère et/ou oligomère (méth)acrylique et au moins un photoinitiateur pour améliorer la tenue en durée de ladite composition pour vernis à ongles tout en proposant un démaquillage facile.

## Description

### Domaine technique

La présente invention concerne de nouvelles compositions cosmétiques applicables sur les ongles en tant que revêtement de base formant un film tenace avec une adhésion supérieure.

### Etat de la technique

Les vernis à ongles sont généralement utilisés pour maquiller les ongles ainsi que les faux ongles. Les vernis à ongles dits «conventionnels» sont habituellement composés d'un agent filmogène, d'un solvant, et éventuellement d'un ou plusieurs plastifiants, d'une ou plusieurs résines, d'un ou plusieurs agents de rhéologie et un ou plusieurs colorants. Les compositions de vernis à ongles peuvent s'appliquer aussi bien sur des ongles naturels que sur des faux-ongles.

Les compositions pour vernis à ongles peuvent être employées comme revêtement de base pour vernis (aussi appelée *base-coat* selon la terminologie anglo-saxonne), comme produit de maquillage des ongles proprement dit, ou comme composition de finition (aussi appelée *top-coat* selon la terminologie anglo-saxonne). Les compositions dites revêtement de base correspondent à un revêtement que l'on applique directement au contact de l'ongle et/ou du faux-ongle afin de protéger l'ongle et/ou de préparer les ongles et/ou de faciliter la pose et le retrait du vernis. Ces compositions dites de revêtement de base sont généralement incolores mais peuvent être colorées ou pigmentées si un effet particulier est recherché. Les compositions de finition peuvent s'appliquer sur le produit de maquillage des ongles proprement dit. Ces produits de finition sont généralement transparents et brillants, afin d'apporter de la brillance et de la résistance à la couche sous-jacente, mais peuvent néanmoins contenir des éléments de décoration, notamment des glitters si un effet particulier est souhaité.

Il existe dans le domaine des vernis à ongles en principe deux types de compositions qui se distinguent significativement par leur durée pendant laquelle elles peuvent rester sur l'ongle sans se détériorer significativement. Les vernis à ongles dits « conventionnels » (non réticulables) sont les bases dites « laque ou solvant » elles présentent souvent une adhésion de courte durée (typiquement 5 jours), mais on peut les retirer aisément. D'un autre côté, il existe les bases dites «gels UV» ou encore dites «soak-off» appliquées en institut spécialisé (uniquement professionnel) qui sont caractérisées par une très forte adhésion (3 semaines), mais peuvent induire des dommages importants de l'ongle (peeling) lors de leur retrait.

Les compositions dites « gels UV » sont des compositions liquides qui sous l'action d'un rayonnement lumineux provoquent des réactions de polymérisation et/ou de réticulation *in situ* aboutissant à des réseaux polymériques le plus souvent réticulés. De telles compositions photoréticulables sont communément appelées «gels UV».

Ce type de vernis à ongles est essentiellement constitué par des oligomères acryliques (de types acrylate, méthacrylate ou poly(éthylène glycol), de pigments, d'un photoinitiateur et de quantités faibles de solvant. Si la composition polymérique peut varier, leur modalité d'application reste la même. Sous l'effet d'un rayonnement UV, les photoinitiateurs amorcent un réaction de polymérisation des monomères et oligomères acryliques déposés sur l'ongle pour former un réseau polymérique complexe encapsulant les charges colorées et autres constituants particulaires. Il en résulte un film synthétique fin, compact et brillant capable de tenir plusieurs semaines sur l'ongle. L'intensité de réticulation est ajustée en fonction de la fonctionnalité du dépôt. Ainsi, la conception de faux ongles ou d'élongation de nature acrylique nécessite une densité de réticulation supérieure à un simple dépôt d'un vernis acrylique coloré. L'exceptionnelle adhésion et cohésion du film sur l'ongle sont dues d'une part à la densité du maillage polymérique formé combiné à une diffusion partielle des oligomères et monomères acryliques à la surface de l'ongle conduisant à une encapsulation partielle de la fibre de kératine présente à la surface de l'ongle. De ce fait, ce type de coating est généralement appliqué dans des instituts spécialisés à même de contrôler efficacement les méthodes de dépôt et le temps d'irradiation UV (« UV curing ») qui peuvent varier selon la nature des monomères et oligomères utilisés. Cette adhésion mécanique ou éventuellement chimique à la fibre de kératine peut induire un retrait du coating par peeling. Du fait de leur extrême stabilité, le retrait de ces bases acryliques ne se fait cependant pas sans dommage pour l'ongle. Les dommages induits par le retrait sont directement liés à la quantité de liens acryliques, entourant l'ongle et formés lors de la polymérisation.

Il est à noter qu'il existe sur le marché des gels UV pouvant être appliqués par les utilisateurs eux-mêmes. Pour favoriser leur tenue sur l'ongle, ils nécessitent cependant une étape de dépolissage de l'ongle afin de favoriser la tenue de la composition photoréticulée. De plus, ils nécessitent, lors du démaquillage, une étape de grattage de l'ongle par un outil métallique, une ponceuse électrique, ou une lime abrasive et/ou plonger les ongles pendant 5 à 10 minutes dans des solvants type acétone. Ces étapes peuvent considérablement et durablement endommager l'ongle. Les gels UV nécessitent également une étape de dégraissage pour éliminer l'effet collant de la surface.

Les vernis à ongles dits «conventionnels» (non réticulables) sont les bases dites « laque ou solvant » qu'on peut aisément retirer, mais elles présentent souvent une adhésion de courte durée (typiquement 5 jours). D'un autre côté, les bases dites «gels UV» ou encore dites «soak-off» appliquées en institut spécialisé (uniquement professionnel) sont caractérisées par une très forte adhésion (3 semaines), mais peuvent induire des dommages importants de l'ongle (peeling) lors de leur retrait.

La tenue dans le temps des vernis à ongles constitue donc un enjeu majeur. Les deux paramètres critiques caractérisant cette tenue dans le temps sont l'adhésion du vernis à ongles et la brillance.

Le challenge majeur dans le domaine des compositions pour vernis à ongles reste donc le développement de formulations non toxiques, à tenue de longue durée, mais néanmoins détachables facilement.

En conséquence, il existe un besoin de développer des vernis à ongles longue durée ne présentant pas les inconvénients des «gels UV» déjà présents sur le marché, c'est-à-dire des vernis à ongles ne présentant pas de difficultés lors du retrait et n'induisant pas de dommages à l'ongle. Idéalement ces compositions permettent un maquillage de qualité dont la tenue et la brillance des compositions sont améliorées. En particulier, il existe une réelle demande de fournir des agents d'amélioration de la tenue de compositions dites revêtements de base compatibles avec tous les types de formulations de vernis à ongles, sans altérer (significativement) leurs autres propriétés. De préférence, ces compositions sont facilement démaquillables avec des démaquillants/dissolvants standards, donc sans utilisation d'outils supplémentaires et sont, de plus, faciles à utiliser par les utilisateurs eux-mêmes.

### Objet de l'invention

Un objet de la présente invention est par conséquent de fournir une composition pour vernis à ongles en tant que revêtement de base présentant une meilleure tenue (de longue durée) sur l'ongle tout en maintenant ses autres propriétés, à savoir : une grande facilité d'application, ainsi qu'un temps de séchage et de durcissement courts.

### Description générale de l'invention

Afin de résoudre le problème mentionné ci-dessus, la présente invention propose, dans un premier aspect, une composition en tant que revêtement de base pour les vernis à ongles comprenant au moins un solvant organique cosmétiquement acceptable, au moins un agent filmogène, un système de photoréticulation comprenant au moins un monomère et/ou oligomère (méth)acrylique et au moins un photoinitiateur.

Dans un deuxième aspect, l'invention propose également un kit (un nécessaire).

Dans un troisième aspect, l'invention propose un procédé de préparation et de protection, ainsi qu'un procédé de maquillage des ongles et/ou des faux-ongles.

Dans un quatrième aspect, l'invention concerne aussi l'utilisation d'une composition.

Les inventeurs ont découvert d'une manière surprenante que la combinaison d'une composition dite « conventionnelle » comprenant un agent filmogène, un solvant organique additionnée d'un système de photoréticulation comprenant des oligomères et/ou monomères (méth)acrylique et un ou des photoinitiateurs, permettait d'améliorer très nettement la tenue en durée des compositions cosmétiques après application tout en pouvant être enlevée d'une manière simple comme justement les compositions conventionnelles.

En effet, les vernis à ongles dits « conventionnels » ont comme avantage de s'appliquer et de se démaquiller très facilement et rapidement. Or, ces vernis à ongles s'écaillent très vite. D'un autre côté, les compositions gels UV réticulables présentent, elles, une tenue longue durée mais restent difficiles à enlever avec comme risque de s'abimer les ongles. De plus, en raison de leurs résistances de longue durée, les utilisateurs ne peuvent modifier leur manucure à volonté.

La composition selon l'invention présente donc les avantages suivants:
- tenue de longue durée,
- la composition est facilement et rapidement démaquillable avec des démaquillants standards,
- les utilisateurs peuvent, au gré de leur envie, modifier leur manucure aussi aisément qu'avec des vernis à ongles « conventionnels ».

Une composition n'est pas juste une addition d'ingrédients facilement remplaçable par d'autres et il est probable que l'ajout ou la substitution d'un composant affecte négativement d'autres propriétés de la formulation. Par conséquent, l'avantage de la présente invention consiste à proposer des familles d'ingrédient compatibles entre eux et qui s'influencent mutuellement pour obtenir un effet de tenue sans compromettre l'intégrité de la formulation.

Un avantage additionnel important de la présente invention est que les compositions en tant que revêtement de base pour les vernis à ongles, de préférence les formulations de vernis à ongles, à tenue améliorée peuvent comprendre les ingrédients habituellement utilisés par l'homme de l'art. L'homme de métier n'est donc pas obligé de reconsidérer tous les ingrédients et leurs effets, s'il souhaite utiliser les présents ingrédients d'améliorations de la tenue. Les présents ingrédients d'amélioration de la tenue présentent par conséquent une très bonne compatibilité tant avec les solvants, qu'avec les ingrédients courants dans le domaine.

De façon préférée, la composition en tant que revêtement de base pour les vernis à ongles peut comprendre une quantité de résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique, ou de leurs combinaisons, comprise entre 0 % et 10 % en poids sec et de façon particulièrement préférée de 2 % à 8 % en poids sec par rapport au poids total de la composition.

Les compositions de revêtement de base pour les vernis à ongles comprennent un solvant organique. Ce solvant organique peut être choisi parmi les solvants usuels dans les vernis à ongles « conventionnels », de préférence dans le groupe constitué par le toluène, le xylène, l'acétate d'éthyle, l'acétate de butyle, l'acétate d'isobutyle, l'acétate de propyle, l'acétate de méthyle, une cétone, un ester, l'isopropanol, le butanol, le diacétone alcool, l'éthanol dénaturé (avec la méthyléthylcétone ou le diéthyléther), les hydrocarbures linéaires ou cycliques, comme par exemple l'hexane, le cyclohexane, l'heptane et n'importe quelle combinaison de ceux-ci.

Le solvant organique pourra être choisi en fonction de ses propriétés physico-chimiques et en considérations des autres composants présents dans la composition de revêtement de base pour les vernis à ongles. De façon préférée, la quantité de solvant organique présente dans la composition de revêtement de base pour les vernis à ongles est comprise entre 20 % et 80 % en poids par rapport au poids total de la composition.

Avantageusement, le solvant organique est l'acétate de butyle et/ou l'acétate d'éthyle.

La composition de revêtement de base pour les vernis à ongles selon l'invention comprend également un agent filmogène. Le ou les agent(s) filmogène(s) pouvant être compris dans la composition de revêtement de base pour les vernis à ongles peu(ven)t être choisi(s) parmi le groupe constitué par la cellulose, un dérivé de cellulose, une résine vinylique, une résine acrylique et n'importe quelle combinaison de ceux-ci. Les dérivés de cellulose sont par exemple la nitrocellulose, l'acétate butyrate cellulose, l'éthylcellulose et l'hydroxy¬propyl¬cellulose. Préférentiellement, on choisira la nitrocellulose comme agent filmogène.

Un agent filmogène seul ou une combinaison d'agents filmogènes peut être utilisé(e) dans la composition de revêtement de base pour les vernis à ongles dans les proportions allant de 2 % à 20 % en poids sec et de façon préférée de 5 % à 12 % en poids sec par rapport au poids total de la composition.

La composition de revêtement de base pour les vernis à ongles comprend au moins un monomère et/ou oligomère (méth)acrylique qui est choisi parmi les monomères suivants, respectivement parmi les oligomères comprenant de 2 à 10 unités monomériques dont au moins un des monomères suivants:
- les méthacrylates de formule CH₂ = C(CH₃)-COOR₁,
dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que l'acrylate d'isobornyle ou un groupe tertio butyle,
- les (méth)acrylamides de formule : 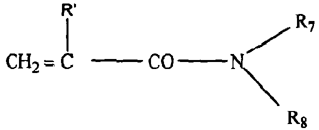
où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges.

La quantité du au moins un monomère et/ou oligomère (méth)acrylique est comprise de 4 % à 40 % en poids sec par rapport au poids total de la composition.

La composition de revêtement de base pour les vernis à ongles comprend également au moins un photoinitiateur qui est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine.

La quantité du au moins un photoinitiateur est comprise de 0,2% à 10% en poids sec par rapport au poids total de la composition.

De manière avantageuse, le système de photoréticulation est utilisé en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons. De préférence, la résine est la résine de copolymère(s) par estérification de l'anhydride phtalique avec le néopentylglycol et/ou le triméthylolpropane et/ou le décanoate de glycidyle comprenant ou est constituée de résine de Polyester-23 (désignation INCI). Par conséquent, la présente invention propose également des compositions de revêtement de base comprenant en outre une ou plusieurs résine(s) choisie(s) parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique, de préférence le Polyester-23.

La composition en tant que revêtement de base pour les ongles peut aussi comprendre au moins un plastifiant. Les plastifiants sont des composés ayant une température d'ébullition élevée qui ne s'évaporent pas lors du séchage du vernis à ongles. Ils sont utilisés pour moduler la dureté du film formé afin d'obtenir les caractéristiques physico-chimiques du film souhaitées et représentent en général de 0 % à 12 % en poids sec, de préférence 4 % à 8 % en poids sec par rapport au poids total de la composition. Le plastifiant est de préférence choisi parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide, etc.

Parfois il est souhaité de colorer ou pigmenter le revêtement de base pour obtenir, par exemple, un effet de profondeur. Par conséquent, pour colorer ou fournir des effets particuliers à la composition de revêtement de base pour les vernis à ongles, celle-ci peut comprendre un ou plusieurs agents de coloration tels que les pigments.

En particulier, les pigments utilisés peuvent être sélectionnés parmi le groupe constitué par le dioxyde de titane (CI 77891), l'oxyde de fer noir (CI 77499), l'oxyde de fer rouge (CI 77491), le DC Red 6 Ba Lake (CI 15850), le DC Red 7 Ca Lake (CI 15850:1), le DC Red 34 Ca Lake (CI 15880), le FDC Yellow 5 Al Lake (CI 19140), le FDC Blue 1 Al Lake (CI 42090), le bleu ultramarine (CI 77007), le DC Violet 2 (CI 60725), le DC Black 2 (CI 77266), etc.

Selon un mode de réalisation de l'invention, la composition de revêtement de base pour les vernis à ongles comprend une quantité d'agent de coloration comprise de 0,1 % à 20 % en poids sec et de préférence de 0,5 % à 14 % en poids sec par rapport au poids total de la composition.

La composition de revêtement de base pour les vernis à ongles peut en outre comprendre divers additifs choisis parmi les agents filmogènes, les résines (autres que celles mentionnées ci-dessus), les modificateurs de surface et les agents dits « traitants ».

Parmi les additifs utilisables selon l'invention, on citera les modificateurs de surface comme les cyclométhicones ou cyclopentasiloxane, les diméthicones et le triméthylsiloxysilicate.

Ces additifs de surface sont généralement utilisés dans des faibles quantités dans la formulation allant de 0,05 à 5 % en poids sec par rapport au poids total de la composition.

On citera également les additifs dits « traitants » de l'ongle utilisables dans la composition selon l'invention. Les additifs traitants peuvent représenter de 0,01 % à 5 % en poids sec, de préférence de 0,05 % à 2 % en poids sec par rapport au poids total de la composition. Parmi ces additifs « traitants », on citera:
- le formaldéhyde utilisé comme durcisseur de l'ongle,
- la vitamine E acétate (tocophéryl acétate),
- la vitamine A palmitate (rétinyl palmitate),
- les dérivés organiques ou inorganiques de calcium tels que le chlorure de calcium, le pantothénate de calcium,
- le diméthyloxobenzodioxasilane,
- la myrrhe,
- les acides aminés soufrés comme la cystéine, ses sels et leurs dérivés, la cystine, le glutathion. Les acides aminés soufrés sont en effet capables de créer des ponts de réticulation entre la kératine et les groupes SH libres de ces dérivés soufrés,
- la kératine hydrolysée d'origine végétale,
- les alpha-hydroxyacides comme l'acide citrique, l'acide ascorbique,
- la biotine,
- l'urée et la diméthylurée.

Un autre objet de l'invention concerne un revêtement de base pour les vernis à ongles comprenant une composition telle que décrite précédemment.

La présente invention concerne un kit comprenant au moins un revêtement de base, un produit de maquillage pour les ongles et (éventuellement) un produit de finition.

Un kit au sens de la présente invention signifie par exemple que les première, deuxième compositions et (éventuellement) la troisième composition sont commercialisées sous un même conditionnement, ou dans deux ou (éventuellement) trois conditionnements séparées sous un même emballage, ou dans deux ou (éventuellement) trois conditionnements séparés sous leur emballage respectif avec une indication d'utilisation combinée de la première composition et de la deuxième composition et (éventuellement) la troisième.

Selon un mode de réalisation particulier, les éléments du kit selon l'invention sont destinés à être appliqués sur les ongles et/ou faux-ongles selon le procédé de l'invention.

Dans un premier aspect, l'invention propose un procédé de préparation et de protection longue durée des ongles et/ou des faux-ongles, facilement démaquillable comprenant au moins les étapes suivantes:
a) application sur un ongle ou un faux-ongle d'une première composition d'un kit conforme à l'invention, par laquelle on dépose un revêtement de base constitué d'au moins une couche de ladite composition, ce revêtement de base étant appliqué directement au contact de l'ongle ou du faux-ongle, et
b) exposition de l'ongle ou du faux-ongle revêtu obtenu à l'issue de l'étape a) à un rayonnement lumineux, UV ou visible.

Dans un deuxième aspect, l'invention propose un procédé de maquillage des ongles et/ou des faux-ongles comprenant au moins les étapes suivantes:
a) application sur un ongle ou un faux-ongle d'une première composition d'un kit conforme à l'invention, par laquelle on dépose un premier revêtement de base constitué d'au moins une couche de ladite composition, ce premier revêtement de base étant appliqué directement au contact de l'ongle ou du faux-ongle, et
b) exposition de l'ongle ou du faux-ongle revêtu obtenu à l'issue de l'étape a) à un rayonnement lumineux, UV ou visible,
c) optionnellement, application sur le premier revêtement de base issu de l'étape a) et b) d'une deuxième composition de maquillage des ongles d'un kit conforme à l'invention, de préférence distincte de la première composition de revêtement de base, par laquelle on dépose un deuxième revêtement constitué d'au moins une couche de ladite deuxième composition, de préférence un produit de maquillage photoréticulable ou non,
d) optionnellement si le deuxième revêtement comprend une composition photoréticulable, exposition de l'ongle ou du faux-ongle revêtu obtenu à l'issu de l'étape c) à un rayonnement lumineux, UV ou visible,
e) optionnellement, application sur le premier ou le deuxième revêtement, d'un troisième revêtement constitué d'au moins une couche d'une troisième composition, de préférence un produit de finition photoréticulable ou non,
f) optionnellement si le troisième revêtement comprend une composition photoréticulable, exposition de l'ongle ou du faux-ongle revêtu obtenu à l'issu de l'étape e) à un rayonnement lumineux, UV ou visible.

Dans un tel procédé, le deuxième revêtement optionnel peut donc aussi être une composition de produit de maquillage photoréticulable ou non, voire une couche de finition photoréticulable ou non, éventuellement exempte d'agent de coloration.

Selon un mode de réalisation, le procédé de l'invention comprend en outre, avant l'étape b), une période de séchage du revêtement déposé à l'issue de l'étape a), dont la durée peut varier de 10 secondes à 10 minutes, typiquement de 30 secondes à 5 minutes. Ledit séchage est généralement effectue à l'air et à la température ambiante. Puis à l'étape b) 10 secondes à 5 minutes sous le rayonnement lumineux, UV ou visible.

Les revêtements réticulés issus de la réticulation de l'étape b) et d) présentent une tenue dans le temps, en termes de résistance à l'écaillement et de brillance, significative et notamment à l'échelle d'au moins 7 à 20 jours. Il s'avère ainsi résistant à l'eau, aux frottements et aux chocs, et ne présente pas d'usure ni d'écaillage significatifs dans ce délai.

Ces revêtements possèdent également une aptitude à se solubiliser ou à augmenter de volume, lorsqu'il est mis en contact avec un solvant de démaquillage usuel. Cette aptitude à se solubiliser ou à gonfler, manifestée par le revêtement réticulé, est précisément avantageuse pour son élimination lorsque celui-ci est appliqué en surface d'un ongle ou d'un faux-ongle. En effet, les revêtements peuvent être aisément éliminés par simple démaquillage à l'aide d'un dissolvant classique.

Un aspect supplémentaire de l'invention concerne, l'utilisation d'un système de photoréticulation comprenant des oligomères et/ou monomères (méth)acrylique et un ou des photoinitiateurs ; de préférence en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons ; pour améliorer la tenue en durée des compositions cosmétiques après application en combinaison avec un démaquillage au dissolvant.

### Exemples

La composition de revêtement de base suivante a été préparée:

| Ingrédient | Quantité (% en poids) |
|---|---|
| acétate d'éthyle | 37,00 |
| acétate de butyle | 30,60 |
| nitrocellulose | 7,00 |
| Alcool isopropylique | 3,00 |
| Polyester-23 | 2,40 |
| (méth)acrylate d'isobornyle | 10,00 |
| PEG-4 trimethylolpropane triacrylate (et) pentaerythrityl tetramercaptopropionate | 9,00 |
| Bis-trimethylbenzoyl phenylphosphine oxide | 1,00 |

La première composition de revêtement de base est appliquée directement sur un ongle sans que celui-ci soit limé.

La composition de revêtement de base réticulable est collante après application, mais le collant disparaît après exposition au rayonnement. On peut directement appliquer la teinte après réticulation.

Un avantage supplémentaire de l'invention est qu'après photoréticulation, la surface des compositions selon l'invention ne nécessite plus d'être nettoyée avec un coton imbibé d'isopropanol afin de retirer la couche collante.

La dureté du revêtement de base est contrôlée en étalant un film fin de 100 µm sur une plaque en verre à l'aide d'un barcoater. La plaque est mise à l'étuve pendant 12h. La dureté est mesurée grâce à un pendule de Persoz. L'essai de dureté au pendule s'appuie sur le principe selon lequel l'amplitude de l'oscillation d'un pendule qui touche une surface diminue plus rapidement si cette surface est tendre. La plaque de verre est bloquée entre le support du pendule et une plaque de métal. La dureté est donc notée en nombre d'oscillations du pendule avant d'atteindre un angle de 4°.

D'un point de vue de la dureté, après application et après 5 minutes de séchage à la lumière naturelle, D=20-40 sec, contre D=80-150 sec après 1 min sous UV. Après ce traitement et 12h passées à l'étuve à 32°C (méthode classique), les duretés de la composition de revêtement de base exposée à la lumière n'ont pas varié, le collant est toujours légèrement présent pour celles exposées à la lumière du jour seulement mais D=250-350 sec pour les compositions de revêtement de base exposées à la LED. Les UV favorisent l'adhésion et la dureté de la composition de revêtement de base.

L'adhésion est bien plus importante que pour une composition de revêtement de base classique due aux monomères et oligomères.

Avec ou sans UV, les compositions de revêtement de base ainsi qu'une couche de base en tant que produit de maquillage pour les ongles classique (non photoréticulable) sont appliqués entre 50 et 100 micromètres pour chacune des 2 couches sur une plaque de verre propre. Selon les normes DIN EN ISO 2409, ASTM D3002 et ASTM D3359, un quadrillage avec un couteau possédant des lames écartées de 1 mm puis application d'un scotch TESA 4124 pendant 1 min 30 est réalisé.

La première adhésion obtenue uniquement par quadrillage donne une adhésion convenable pour la composition de revêtement de base seule ou une composition de revêtement de base classique (non photoréticulable) ou photoréticulable avec une couche de base en tant que produit de maquillage pour les ongles (non photoréticulable) par-dessus.

Cependant, après application du scotch, toute la couche de vernis et du système complet disparaît pour la composition de revêtement de base classique seule ou la composition de revêtement de base classique. Contrairement aux systèmes de composition de revêtement de base photoréticulables en combinaison avec une base classique pour lesquels la composition de revêtement de base ne se décolle pas, la teinte se décolle un peu de la composition base coat mais pas totalement. L'adhésion est bien améliorée. Avec passage sous LED, le film est plus dur et la base semble davantage coller à la composition de revêtement de base qui, elle, reste collée à la plaque.

Les compositions photoréticulables de revêtement de base en combinaison avec une composition de produit de maquillage pour les ongles non photoréticulable présentent une tenue dans le temps, en termes de résistance à l'écaillement et de brillance significative et notamment à l'échelle d'au moins 7 à 20 jours. Les compositions s'avèrent ainsi résistantes à l'usure, à l'écaillement significatif dans ce délai.

Ces compositions se démaquillent avec un solvant de démaquillage usuel. En effet, un avantage de l'invention est que les compositions ne nécessitent plus de solvant abrasif comme l'acétone ou de lime. Le film, après nettoyage avec l'acétate d'éthyle, se décompose et se soulève de son substrat.

## Revendications

1. Composition de revêtement de base pour les vernis à ongles comprenant au moins un solvant organique cosmétiquement acceptable, au moins un agent filmogène et un système de photoréticulation comprenant au moins un monomère et/ou oligomère (méth)acrylique et au moins un photoinitiateur.

2. Composition de revêtement de base pour les vernis à ongles selon la revendication 1, **caractérisée en ce que** le système de photoréticulation y est compris en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons, la au moins une résine étant de préférence la résine de copolymère(s) par estérification de l'anhydride phtalique avec le néopentylglycol et/ou le triméthylolpropane et/ou le décanoate de glycidyle, de manière davantage préférée la résine de copolymère(s) par estérification de l'anhydride phtalique avec le néopentylglycol et/ou le triméthylolpropane et/ou le décanoate de glycidyle comprend ou est constituée de résine de Polyester-23 (désignation INCI).

3. Composition de revêtement de base pour les vernis à ongles selon l'une quelconque des revendications précédentes, **caractérisée en ce que** la quantité de résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/ glycols, les copolymères acide adipique/néopentylglycol/anhydride trimellitique, les copolymères de glycérine/acide phtalique est comprise entre 1 % et 10 % en poids sec et de façon préférée de 2 % à 8 % en poids sec par rapport au poids total de la composition.

4. Composition de revêtement de base pour les vernis à ongles selon l'une quelconque des revendications précédentes, comprenant au moins un solvant organique choisi parmi le toluène, le xylène, l'acétate d'éthyle, l'acétate de butyle, l'acétate d'isobutyle, l'acétate de propyle, l'acétate de méthyle une cétone, un ester, l'isopropanol, le butanol le diacétone alcool, l'éthanol dénaturé avec la méthyléthylcétone ou le diéthyléther, les hydrocarbures linéaires ou cycliques, de préférence le cyclohexane, l'heptane ou n'importe quelle combinaison de ceux-ci, de préférence le solvant organique est l'acétate d'éthyle, l'acétate de butyle ou une combinaison de ceux-ci, la quantité de solvant organique étant de préférence comprise entre 20 % et 80 % en poids par rapport au poids total de la composition pour vernis à ongles.

5. Composition de revêtement de base pour les vernis à ongles selon l'une quelconque des revendications précédentes, **caractérisée en ce que** le au moins un monomère et/ou oligomère (méth)acrylique est choisi parmi les monomères suivants, respectivement parmi les oligomères comprenant au moins un des monomères suivants:
- les méthacrylates de formule CH₂ = C(CH₃)-COOR₁,
dans laquelle R₁, représente un groupe alkyle non substitué, linéaire ou ramifié, contenant de 1 à 4 atomes de carbone, tel qu'un groupe méthyle, éthyle, propyle ou isobutyle ou R₁ représente un groupe cycloalkyle C₄ à C₁₂,
- les acrylates de formule CH₂ = CH-COOR₂
dans laquelle R₂ représente un groupe cycloalkyle en C₄ à C₁₂ tel que l'acrylate d'isobornyle ou un groupe tertio butyle,
- les (méth)acrylamides de formule : 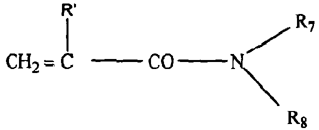 où R₇ et R₈ identiques ou différents représentent chacun un atome d'hydrogène ou un groupe alkyle de 1 à 12 atomes de carbone linéaire ou ramifié, tel qu'un groupe n-butyle, t-butyle, isopropyle, isohexyle, isooctyle, ou isononyle ; où R₇ représente H et R₈ représente un groupement 1,1-diméthyl-3-oxobutyl, et R' désigne H ou méthyle, et leurs mélanges,
la quantité du au moins un monomère et/ou oligomère (méth)acrylique étant de préférence comprise de 4 % à 40 % en poids sec par rapport au poids total de la composition.

6. Composition de revêtement de base pour les vernis à ongles selon l'une quelconque des revendications précédentes, **caractérisée en ce que** le au moins un photoinitiateur est choisi parmi le groupe constitué des α-hydroxycétones, des α-aminocétones, des cétones aromatiques de préférences associées à un composé donneur d'hydrogène, des α-dicétones aromatiques et des oxydes d'acylphosphine, et de leurs mélanges, avantageusement dans le groupe constitué des oxydes d'acylphosphine, la quantité du photoinitiateur étant de préférence comprise de 0,2% à 10% en poids sec par rapport au poids total de la composition.

7. Composition de revêtement de base pour les vernis à ongles selon l'une quelconque des revendications précédentes, **caractérisée en ce que** l'agent filmogène est choisi parmi la cellulose, un dérivé de cellulose, une résine vinylique, ou n'importe quelle combinaison de ceux-ci, de préférence l'agent filmogène est la nitrocellulose, la quantité d'agent filmogène étant de préférence comprise de 2 % à 20 % en poids sec et de façon davantage préférée de 5 % à 12 % en poids sec par rapport au poids total de la composition.

8. Composition de revêtement de base pour les vernis à ongles selon l'une quelconque des revendications précédentes, comprenant en outre un ou plusieurs plastifiants choisis parmi l'acétyl tributyl citrate, l'acétyl triéthyl citrate, le tributyl citrate, le triéthylcitrate, le dibutyl phtalate, le triphénylphosphate, la triacétine, le triméthyl pentanyl diisobutyrate, la triéthylhexanoïne, le sucrose benzoate, le dibutyl adipate, le diéthyl phtalate, le diisobutyl adipate, le diisopropyl adipate, le dipropylène glycol dibenzoate, le N-éthyl toluène sulfonamide, ses isomères ortho et para, le N-(2-hydroxypropyl) benzène sulfonamide et le N-(n-butyl) benzène sulfonamide, ou leurs combinaisons, le ou les plastifiant(s) représentant de préférence de 0 % à 12 % en poids sec, de façon davantage préférée de 4 % à 8 % en poids sec par rapport au poids total de la composition.

9. Composition de revêtement de base pour les vernis à ongles selon l'une quelconque des revendications précédentes, comprenant en outre un ou plusieurs additifs choisis parmi les résines, les modificateurs de surface et les agents dits « traitants ».

10. Composition de revêtement de base pour les vernis à ongles selon l'une quelconque des revendications précédentes, **caractérisée en ce qu'**elle comprend en outre un ou plusieurs agents de coloration, tels que les pigments.

11. Revêtement de base pour les vernis à ongles comprenant une composition selon l'une quelconque des revendications précédentes.

12. Kit comprenant une composition de revêtement de base selon l'une quelconque des revendications 1 à 10 ou un revêtement de base selon la revendication 11 et un produit de maquillage pour les ongles et éventuellement un produit de finition.

13. Procédé de préparation et de protection longue durée des ongles et/ou des faux-ongles, facilement démaquillable comprenant les étapes suivantes:
a) application sur un ongle ou un faux-ongle d'une première composition de revêtement de base selon l'une quelconque des revendications 1 à 10 ou d'un d'un kit selon la revendication 12, par laquelle on dépose un revêtement de base constitué d'au moins une couche de ladite composition, ce revêtement de base étant appliqué directement au contact de l'ongle ou du faux-ongle, et
b) exposition de l'ongle ou du faux-ongle revêtu obtenu à l'issue de l'étape a) à un rayonnement lumineux, UV ou visible.

14. Procédé de maquillage des ongles et/ou des faux-ongles facilement démaquillable comprenant les étapes suivantes:
a) application sur un ongle ou un faux-ongle d'une première composition de revêtement de base selon l'une quelconque des revendications 1 à 10 ou d'un d'un kit selon la revendication 12, par laquelle on dépose un premier revêtement de base constitué d'au moins une couche de ladite composition, ce premier revêtement de base étant appliqué directement au contact de l'ongle ou du faux-ongle, et
b) exposition de l'ongle ou du faux-ongle revêtu obtenu à l'issue de l'étape a) à un rayonnement lumineux, UV ou visible,
c) optionnellement, application sur le premier revêtement de base issu de l'étape a) et b) d'une deuxième composition de maquillage des ongles, de préférence distincte de la première composition de revêtement de base, par laquelle on dépose un deuxième revêtement constitué d'au moins une couche de ladite deuxième composition, de préférence un produit de maquillage photoréticulable ou non,
d) optionnellement si le deuxième revêtement comprend une composition photoréticulable, exposition de l'ongle ou du faux-ongle revêtu obtenu à l'issu de l'étape c) à un rayonnement lumineux, UV ou visible,
e) optionnellement, application sur le premier ou le deuxième revêtement issu des étapes a) à d), d'un troisième revêtement constitué d'au moins une couche d'une troisième composition, de préférence un produit de finition photoréticulable ou non,
f) optionnellement si le troisième revêtement comprend une composition photoréticulable, exposition de l'ongle ou du faux-ongle revêtu obtenu à l'issu de l'étape e) à un rayonnement lumineux, UV ou visible.

15. Utilisation d'un système de photoréticulation comprenant des oligomères et/ou monomères (méth)acrylique et un ou des photoinitiateurs ; de préférence en association avec au moins une résine choisie parmi une résine de copolymère(s) par estérification de l'anhydride phtalique avec le néopentylglycol et/ou le triméthylolpropane et/ou le décanoate de glycidyle, les copolymères d'anhydride phtalique/glycérine/ décanoate de glycidyle, les copolymères d'anhydride phtalique/anhydride trimellitique/glycols, les copolymères acide adipique/néopentylglycol/anhydride trimellitique, les copolymères de glycérine/acide phtalique et leurs combinaisons ; pour améliorer la tenue en durée des compositions cosmétiques après application en combinaison avec un démaquillage au dissolvant.
